# EUROPEAN PATENT APPLICATION

(11) **EP 1 459 767 A1**
(43) Date of publication of application: **22.09.2004**
(21) Application number: 02790886.2
(22) Date of filing: 26.12.2002
(51) Int. Cl.: A61K 47/38, A61K 9/48, A61K 47/32, A61K 47/36, A61K 35/78, A61P 3/02

(54) **HARD CAPSULES**

(30) Priority: 28.12.2001 JP 2001400903
(71) Applicant: WAKUNAGA PHARMACEUTICAL CO., LTD., Osaka-shi, Osaka 532-0003 (JP)
(72) Inventor: MOTOUNE, Soko WAKUNAGA PHARMACEUTICAL CO., LTD., TAKATA-GUN, Hiroshima 739-1195 (JP); IKEDA, Yoichi WAKUNAGA PHARMACEUTICAL CO., LTD., TAKATA-GUN, Hiroshima 739-1195 (JP)
(74) Representative: Hartz, Nikolai F., Dr.
(86) International application number: PCT/JP2002/013574
(87) International publication number: WO 2003/057256

(57) **Abstract**

The present invention is directed to a hard capsule filled with a solution containing an active ingredient, wherein the shell of the capsule is made of a material containing a cellulose derivative, and the solution has a water content (w) of 10% < w ≤ 50%, and a water activity (a) of 0.60 ≤ a ≤ 0.90.

The present invention provides a hard capsule which can hold an active-ingredient-containing solution of high water content, without impairing drug properties and stability, and sensation upon intake or eating sensation.

## Description

### TECHNICAL FIELD

The present invention relates to hard capsules useful as medical drugs, quasi-drugs, cosmetics, and foods, and more particularly to stable hard capsules filled with components of high water content.

### BACKGROUND ART

In the medical and food appliance, capsules have conventionally been employed as suitable for improving the compliance upon use. In particular, hard capsules can be produced easily and therefore have been widely used for containing solid substances such as powder, granules, and fine granules, or oily substances.

Hard capsules typically contain gelatin as a shell-forming material, and therefore, they have high solubility in water. When an active component of high water content is directly charged in such a hard capsule, the internal solution renders the gelatin shells dissolved or moistened, permitting deformation of the shape of the capsules or leakage of the internal solution. As a result, the capsule has no value as a marketable product. Therefore, when the material to be encapsulated contains an aqueous solution, conventional approaches to avoiding the effect of the water include addition of an excipient and solidification through, for example, concentration or drying, performed before encapsulation. In the case where a material is desired to be charged in capsules with its liquidity being maintained, the material is suspended or emulsified in an oily substance before being encapsulated. Alternatively, glycerol is added to the material and the resultant mixture is heated to evaporate the water so as to attain a water content of 10% or less, followed by filling in capsules formed of a cellulose derivative (U.S. Patent No. 6,238,696).

However, when subjected to a solidification process with heat, an animal or plant extract, such as a medicinal herb extract, is prone to change its intrinsic taste, odor, properties, and components. Moreover, when hard capsules containing such an extract are produced by a means of solidification through addition of an excipient, formation of suspension or emulsification in an oily base, or reduction of a water content to 10% or less (which is attained by adding glycerol in an amount as high as 20% to 80% on a final product basis, followed by heating), drawbacks such as limitation being imposed on the amount of the active ingredient incorporated and a rise in production cost are unavoidable.

Meanwhile, soft capsules have been known to be able to contain liquid ingredients. In the case of soft capsules, encapsulation of ingredients of high water content, such as medicinal herb extracts, requires certain techniques. For example, a moisture-containing plant extract and its equivalent similar substance need to be emulsified with a fatty acid glyceride to thereby form soft capsules (Japanese Patent Application Laid-Open (*kokai*) No. 52-35178), or, in order to impart water resistance to a gelatin shell, a layered structure of gelatin sheets and polysaccharide sheets need to be employed in producing soft capsules (Japanese Patent Application Laid-Open (kokai) No. 63-164858). These measures make production steps and production conditions intricate, raising a concern of increased cost.

Therefore, there has been a need for a technique capable of, through simple production steps and at low cost, encapsulating an aqueous extract of medicinal herbs, an aqueous extract of animal or plant origin, or a similar substance without permitting volatilization or degradation of active ingredients, while the resultant capsule products do not impair sensation (taste, odor, etc.) upon intake.

An object of the present invention is to provide hard capsules capable of stably enclosing a solution containing an active ingredient of high water content, such as an aqueous extract of medicinal herbs, an aqueous extract of animal or plant origin, or a similar substance, without impairing its quality for a prolonged period of time.

### DISCLOSURE OF THE INVENTION

In view of the foregoing, the present inventors have performed extensive studies on the relation between properties of the capsule shell and water content of the solution to be encapsulated, and have found that a high-water-content solution can be successfully encapsulated in hard capsules if a specific capsule shell is employed and the water content and the water activity of the solution to be encapsulated can be properly adjusted. The present invention has been achieved on the basis of this finding.

Accordingly, the present invention provides a hard capsule filled with a solution containing an active ingredient, wherein the shell of the capsule is made of a material containing a cellulose derivative, the moisture content (w) of the solution to be encapsulated is 10% < w ≤ 50%, and the water activity (a) is 0.60 ≤ a ≤ 0.90.

### BEST MODE FOR CARRYING OUT THE INVENTION

The solution to be enclosed in the hard capsule of the present invention (hereinafter referred to as "internal solution") is a solution containing an active ingredient, and has a water content (w) of 10% < w ≤ 50% and a water activity (a) of 0.60 ≤ a ≤ 0.90.

As used herein, the term "water activity (a)" of a water-containing medium is defined as the ratio P/P₀ of water vapor pressure of the medium (P) to vapor pressure of pure water (P₀) under the conditions where both pressures are measured at the same temperature. The water activity is a parameter representing activity of water in a medium.

The internal solution is not limited to aqueous solutions of active ingredients, but encompasses suspensions and O/W-type emulsions, as in the case where a portion of active ingredients is difficult to dissolve in water. No particular limitation is imposed on the active ingredients, and they may be compounds or compositions of arbitrary substances, such as medical drugs, natural materials, foods, herbal medicines in the form of extracts, plant extracts, and fermented matter.

Examples of the internal solution include aqueous extracts prepared by subjecting medicinal herbs, animal- or plant-originating substances, or fermented matter to extraction with water or hydrated alcohol. Specific examples falling under this category include herbal medicines in the form of aqueous extracts, such as ginseng, garlic, *Acanthopanax senticosus Harms, Angelica sinensis, Rehmammia glutionosa, Citrus reticulata, Cuscuta chinensis, Schizandra chinesis*, and *Ophioposen japonicus*; aqueous extracts of herbal mixtures employed in *kanpo* (traditional Chinese medicine), available under the names of *kakkon-to, bakumonto-to, sho-seiryu-to, orengedoku-to, shimotsu-to*, and *shakuyakukanzo-to;* aqueous extracts of animal- or plant-origin, prepared from, for example, blue berries, green tea leaves, herbs, mushrooms, and *mamushi* (Japanese copperhead); and aqueous extracts of fermented matter produced by fermenting grains, plants, or marine products with microorganisms such as *koji* mold, *beni-koji* mold, lactic acid bacteria, acetic acid bacteria, *Bacillus natto*, and yeast. Other examples of the internal solution include solutions in water of aqueous vitamins such as vitamin B1, vitamin B2, niacin, vitamin B6, vitamin B12, vitamin C, pantothenic acid, biotin, folic acid, and pantothenyl alcohol; solutions in water of dextromethorphan hydrobromide, acetaminophen, chlorphenylamine maleate, potassium guaiacolsulfonate, caffeine, dihydrocodeine phosphate, methylephedrine hydrochloride, and water-soluble azulene; and suspensions of aldioxa, magnesium hydroxide, sucralfate, magnesium aluminometasilicate, and synthetic hydrotalcite. In particular, herbal medicines in the form of aqueous extracts and aqueous extracts of animal- or plant-origin are preferred, because they are stable as aqueous solutions, and they can be handled in their solution form during the capsule formulation process.

If desired, the above-described internal solution may contain other ingredients, such as a sweetener, an acidulant, a stabilizer, a thickener, a pH regulator, a preservative, a colorant, and a flavoring agent, which are generally used in the production of foods and drugs.

Here, examples of the sweetening agent include sugars such as sucrose, lactose, fructose, and glucose; sugar alcohols such as sorbitol, erythritol, mannitol, xylitol, and trehalose; glycyrrhizin; aspartame; and stevia. These may be used singly or in combination of two or more species.

As the acidulant, those commonly employed in the production of drugs and foods may be used. Examples of acidulants include citric acid, malic acid, succinic acid, fumaric acid, tartaric acid, and lactic acid. These may be used singly or in combination of two or more species.

Examples of the stabilizer include antioxidants such as ascorbic acid, erythorbic acid, and sodium pyrosulfite; dispersants such as sodium pyrophosphate, sodium polyphosphate, and sodium metaphosphate; surfactants such as sucrose fatty acid esters, polyoxyethylene hydrogenated castor oil, and polyoxyethylene polyoxypropylene glycol; cyclodextrins such as cyclodextrin, glucosyl-cyclodextrin, maltosyl-cyclodextrin, and hydroxypropyl-cyclodextrin. These may be used singly or in combination of two or more species.

Examples of the thickener include polymers such as dextrin, sodium alginate, propylene glycol alginate, tragacanth powder, xanthan gum, carrageenan, agar, pectin, carboxymethylcellulose-Na, hydroxypropyl cellolose, polyvinyl alcohol, and polyvinyl pyrrolidone. These may be used singly or in combination of two or more species.

As for the pH regulator, those generally employed in the shape-imparting art may be used. Examples thereof include organic and inorganic acids such as hydrochloric acid, acetic acid, phosphoric acid, citric acid, malic acid, succinic acid, fumaric acid, tartaric acid, lactic acid, and salts of any of these acids, alkalis such as sodium hydroxide, potassium hydroxide, sodium hydrogencarbonate, and sodium dihydrogenphosphate. These may be used singly or in combination of two or more species.

Examples of preservatives include benzoic acids, sorbic acids, p-hydroxybenzoic esters, and salicylic acids. These may be used singly or in combination of two or more species.

Examples of the colorant include caramel, sodium copper chlorophyllin, riboflavin sodium phosphate, indigocarmine, Brilliant Blue, tartrazine, sunset yellow, new coccine, amaranth, and erythrosine. These may be used singly or in combination of two or more species.

As for the flavoring agents, those generally employed for the manufacture of drugs and foods may be used, including, for example, fennel oil, orange oil, cinnamon oil, orange peel oil, mentha oil, vanillin, and eucalyptus oil. In addition to these, there may be employed natural-originating flavors and compounded flavors which are produced using any of these materials as raw material.

The foregoing internal solutions may be adjusted so as to be 10 < w ≤ 50% in the water content (w) and 0.60 ≤ a ≤ 0.90 in the water activity (a), respectively. Preferably, the water content is 15 ≤ w ≤40% and the water activity is 0.60 ≤ a ≤ 0.85.

When the water content of the internal solution exceeds 50% or the water activity exceeds 0.90, water is present in an amount sufficient to dissolve capsule shells, and therefore, these ranges should be avoided. Meanwhile, a water content less than 10% or a water activity below 0.60 should also be avoided, because under such conditions, the viscosity of the internal solution extremely increases and prevents smooth flow of the solution, so that it becomes difficult to fill the shells during production of capsules.

No particular limitation is imposed on the methods of adjusting water content (w) or water activity (a). For example, either of the following 1) or 2) may be performed: 1) Soluble solid matter is caused to dissolve in cold water or lukewarm water until a target water content and a target water activity are achieved, 2) in the case where an animal- or plant-derived crude drug in the aqueous extract form is employed, the relevant animal or plant material is extracted with water or a water-ethanol mixture, and the resultant filtrate is concentrated with the application of heat (25 to 50°C) under reduced pressure until a target water content and a target water activity are achieved.

The capsule shells of the hard capsules according to the present invention are made of a material containing a cellulose derivative.

Examples of the cellulose derivative include hydroxypropyl methylcellulose, methylcellulose, hydroxypropyl cellulose, hydroxyethyl cellulose, hydroxypropyl methylcellulose phthalate, hydroxypropyl methylcellulose acetate succinate, carmelose, carboxymethylethyl cellulose, cellulose acetate phthalate, and ethylcellulose. In particular, hydroxypropyl methylcellulose and methylcellulose are preferred. The cellulose derivative may be used singly or in combination of two or more species.

The cellulose derivative is incorporated in an amount of 15 to 30% by weight on the basis of the aqueous shell solution employed during the production of capsule shells. Also, like the case of the internal solution, additives which are generally employed in the shape-imparting art, such as a gelling agent, a gelling aid, a colorant, a plasticizer, an emulsifier, a dispersant, and a preservative may be added.

Examples of the gelling agent include carrageenan, xanthan gum, locust bean gum, gellan gum, gum arabic, guar gum, tamarind-seed-derived polysaccharides, pectin, curdlan, gelatin, furcellaran, and agar. Preferably, carrageenan is employed. These materials may be used singly or in combination of two or more species. The amount of the gelling agent to be added is 0.1 to 1.0% by weight on the basis of the aqueous shell solution employed for the production of capsule shells.

Examples of the gelling aid include water-soluble compounds which release calcium ions, potassium ions, ammonium ions, sodium ions, or magnesium ions, and specific examples include calcium chloride, potassium chloride, ammonium chloride, ammonium acetate, potassium phosphate, potassium citrate, sodium chloride, magnesium sulfate, and organic acids and water soluble salts thereof (for example, citric acid or sodium citrate). Water-soluble compounds providing potassium ions or ammonium ions are preferably employed. The amount of the gelling aid to be added is 0.01 to 1.0% by weight on the basis of the aqueous shell solution employed for the production of capsule shells.

When a colorant is incorporated, any of ordinarily available additives in the production of capsule shells may be used in suitable amounts. Examples of the colorant include caramel, sodium copper chlorophyllin, riboflavin sodium phosphate, indigocarmine, Brilliant Blue, tartrazine, sunset yellow, new coccine, amaranth, erythrosine, titanium oxides, and iron oxides. These may be used singly or in combination of two or more species.

Examples of the plasticizer include triethyl citrate, triacetin, glycerol, D-sorbitol, polyethylene glycol, acetylated monoglyceride, and surfactants, and these may be used singly or in combination of two or more species.

Moreover, if necessary, the hard capsules of the present invention may be coated with a coating base, or two or more coating bases in combination, containing, for example, hydroxypropyl methylcellulose phthalate, hydroxypropyl methylcellulose acetate succinate, carboxymethylethyl cellulose, cellulose acetate phthalate, polyvinylacetal diethylaminoacetate, or a methacrylate copolymer.

The hard capsules according to the present invention can be produced as follows. As described above, the water content (w) and the water activity (a) of an internal solution are adjusted to 10 < w ≤ 50% and 0.60 ≤ a ≤ 0.90, respectively, and the resultant solution is filled into the aforementioned capsules by use of a conventional capsule filling machine designed to fill oily substances. Thereafter, in each capsule, the joint portion of the capsule body and its corresponding cap is sealed with an aqueous ethanol solution of a cellulose derivative, followed by drying with air.

Packaging of the thus-produced capsules may be performed in any conventional mode, by the use of, for example, bottles, aluminum foil, PTPs, etc.

Preferred examples of the hard capsule of the present invention include those having the property that the internal solution is a medicinal herb extract having a water content (w) of 33% ≤ w ≤ 38% and a water activity (a) of 0.79 ≤ a ≤ 0.82, and the capsule shell contains hydroxypropyl methylcellulose, a gelling agent, and a gelling aid. In particular, a capsule which is #1 to #00 in its capsule size and packed in bottles or aluminum pouches is preferred.

### EXAMPLES

The present invention will next be described in more detail by way of examples, which should not be construed as limiting the invention thereto.

### Example 1

A ginseng extract which had been concentrated under reduced pressure with application of heat at 30°C so as to have a water content falling within a range of 32 to 48% and a water activity falling within a range of 0.80 to 0.89 was filled in #0 capsules made of hydroxypropyl methylcellulose, and in each capsule the joining portion of the capsule receptacle and its corresponding cap was sealed with an aqueous ethanol solution of hydroxypropyl methylcellulose, followed by drying with air. The resultant capsule samples were filled in glass bottles, and stored at 40°C for 4 weeks. Whether there had been any leakage of the internal solution from the capsules was checked by visual observation. The results are shown in Table 1. As a result, no liquid leakage was visually observed in respect of samples corresponding to a water content of 39% or less and a water activity of 0.86 or less during the 4 weeks' storage.

**Table 1**

| Water content (%) | 32 | 39 | 48 |
|---|---|---|---|
| Water activity | 0.80 | 0.86 | 0.89 |
| 1 week | ○ | ○ | ○ |
| 2 weeks | ○ | ○ | × |
| 3 weeks | ○ | ○ | × |
| 4 weeks | ○ | ○ | × |
| ○: No leakage of water ×: Leakage of water | | | |

### Example 2

In a manner similar to that described in Example 1, a garlic extract whose water content and water activity had been adjusted to fall within the ranges of 32 to 49% and 0.73 to 0.87, respectively, was filled in #0 capsules made of hydroxypropyl methylcellulose, followed by sealing and drying with air, as in Example 1. The resultant capsule samples were filled in glass bottles, and stored at 40°C for 4 weeks. A visual observation was performed to determine whether or not the internal solution leaks from each capsule. As shown in Table 2, no liquid leakage was visually observed in respect of samples corresponding to a water content of 41% or less and a water activity of 0.83 or less during the 4 weeks' storage.

**Table 2**

| Water content (%) | 32 | 41 | 49 |
|---|---|---|---|
| Water activity | 0.73 | 0.83 | 0.87 |
| 1 week | ○ | ○ | ○ |
| 2 weeks | ○ | ○ | × |
| 3 weeks | ○ | ○ | × |
| 4 weeks | ○ | ○ | × |
| ○: No leakage of water ×: Leakage of water | | | |

### Example 3

In a manner similar to that described in Example 1, a *kakkon-to* (which is a decoction of roots of *kudzu* or *Pueraria lobata Ohwi*) extract whose water content and water activity had been adjusted to fall within the ranges of 33 to 47% and 0.79 to 0.87, respectively, was filled in #0 capsules made from hydroxypropyl methylcellulose, followed by sealing and drying with air, as in Example 1. The resultant capsule samples were filled in glass bottles, and stored at 40°C for 4 weeks. A visual observation was performed to determine whether or not the internal solution leaks from each capsule. As shown in Table 3, no liquid leakage was visually observed in respect of samples corresponding to a water content of 38% or less and a water activity of 0.82 or less during the 4 weeks' storage.

**Table 3**

| Water content (%) | 33 | 38 | 47 |
|---|---|---|---|
| Water activity | 0.79 | 0.82 | 0.87 |
| 1 week | ○ | ○ | × |
| 2 weeks | ○ | ○ | × |
| 3 weeks | ○ | ○ | × |
| 4 weeks | ○ | ○ | × |
| ○: No leakage of water ×: Leakage of water | | | |

### Example 4

A fructose-glucose syrup whose water content and water activity had been adjusted to 26% and 0.73, respectively, was filled in #0 capsules made from hydroxypropyl methylcellulose, followed by sealing and drying with air, as in Example 1. The resultant capsule samples were filled in glass bottles, and stored at 40°C for 4 weeks. A visual observation was performed to determine whether or not the internal solution leaks from each capsule. As shown in Table 4, the samples showed no liquid leakage during the 4 weeks' storage.

**Table 4**

| Water content (%) | 26 | 26 |
|---|---|---|
| Water activity | 0.73 | 0.73 |
| PH | 3.0 | 4.6 |
| 1 week | ○ | ○ |
| 2 weeks | ○ | ○ |
| 3 weeks | ○ | ○ |
| 4 weeks | ○ | ○ |
| o: No leakage of water ×: Leakage of water | | |

### Example 5

In a manner similar to that described in Example 1, a garlic extract whose water content and water activity had been adjusted to fall within the ranges of 32 to 60% and 0.73 to 0.91, respectively, was filled in previously prepared methylcellulose capsules. Each capsule was sealed at its upper portion with an aqueous ethanol solution of methylcellulose, followed by drying with air. The resultant capsule samples were filled in glass bottles, and stored at 40°C for 4 weeks. A visual observation was performed to determine whether or not the internal solution leaks from each capsule. As shown in Table 5, no liquid leakage was visually observed in respect of samples corresponding to a water content of 50% or less and a water activity of 0.87 or less during the 4 weeks' storage. On the other hand, samples of 60% water content showed water droplets on capsule surfaces, but its shape remained intact.

**Table 5**

| Water content (%) | 32 | 40 | 50 | 60 |
|---|---|---|---|---|
| Water activity | 0.73 | 0.82 | 0.87 | 0.91 |
| 1 week | ○ | ○ | ○ | × |
| 2 weeks | ○ | ○ | ○ | × |
| 3 weeks | ○ | ○ | ○ | × |
| 4 weeks | ○ | ○ | ○ | × |
| o: No leakage of water ×: Leakage of water | | | | |

### Comparative Example 1

A ginseng extract which had been concentrated under reduced pressure with application of heat at 30°C so as to have a water content falling within a range of 32 to 48% and a water activity falling within a range of 0.80 to 0.89 was filled in #0 capsules made from gelatin, and in each capsule the joining portion of the capsule receptacle and its corresponding cap was sealed with an aqueous solution of gelatin, followed by drying with air. The resultant capsule samples were filled in glass bottles, and stored at 40°C for 4 weeks. Whether there had been any leakage of the internal solution from each capsule was checked by visual observation. The results are shown in Table 6. After one week of storage, the liquid began to leak from each of the samples as shown in this table.

**Table 6**

| Water content (%) | 32 | 39 | 48 |
|---|---|---|---|
| Water activity | 0.80 | 0.86 | 0.89 |
| 1 week | × | × | × |
| 2 weeks | × | × | × |
| 3 weeks | × | × | × |
| 4 weeks | × | × | × |
| o: No leakage of water ×: Leakage of water | | | |

### Comparative Example 2

In a manner similar to that described in Comparative Example 1, a garlic extract whose water content and water activity had been adjusted to fall within the ranges of 32 to 49% and 0.73 to 0.87, respectively, was filled in #0 capsules made from gelatin, followed by sealing and drying with air, as in Comparative Example 1. The resultant capsule samples were filled in glass bottles, and stored at 40°C for 4 weeks. A visual observation was performed to determine whether or not the internal solution leaks from each capsule. The results are shown in Table 7. After one week of storage, the liquid began to leak from each of the samples as shown in this table.

**Table 7**

| Water content (%) | 32 | 41 | 49 |
|---|---|---|---|
| Water activity | 0.73 | 0.83 | 0.87 |
| 1 week | × | × | × |
| 2 weeks | × | × | × |
| 3 weeks | × | × | × |
| 4 weeks | × | × | × |
| o: No leakage of water ×: Leakage of water | | | |

### Comparative Example 3

In a manner similar to that described in Comparative Example 1, a *kakkon-to* (which is a decoction of roots of kudzu or *Pueraria lobata Ohwi*) extract whose water content and water activity had been adjusted to fall within the ranges of 33 to 47% and 0.79 to 0.87, respectively, was filled in #0 capsules made from gelatin, followed by sealing and drying with air, as in Comparative Example 1. The resultant capsule samples were filled in glass bottles, and stored at 40°C for 4 weeks. A visual observation was performed to determine whether or not the internal solution leaks from each capsule. The results are shown in Table 8. After one week of storage, the liquid began to leak from each of the samples as shown in this table.

**Table 8**

| Water content (%) | 33 | 38 | 47 |
|---|---|---|---|
| Water activity | 0.79 | 0.82 | 0.87 |
| 1 week | × | × | × |
| 2 weeks | × | × | × |
| 3 weeks | × | × | × |
| 4 weeks | × | × | × |
| ○: No leakage of water ×: Leakage of water | | | |

### Comparative Example 4

A fructose glucose syrup whose water content and water activity had been adjusted to 26% and 0.73, respectively, was filled in #0 capsules made from gelatin, followed by sealing and drying with air, as in Comparative Example 1. The resultant capsule samples were filled in glass bottles, and stored at 40°C for 4 weeks. A visual observation was performed to determine whether or not the internal solution leaks from each capsule. The results are shown in Table 9. After one week of storage, the liquid began to leak from each of the samples as shown in this table.

**Table 9**

| water content (%) | 26 | 26 |
|---|---|---|
| water activity | 0.73 | 0.73 |
| PH | 3.0 | 4.6 |
| 1 week | × | × |
| 2 weeks | × | × |
| 3 weeks | × | × |
| 4 weeks | × | × |
| o: No leakage of water ×: Leakage of water | | |

### INDUSTRIAL APPLICABILITY

The present invention provides hard capsules containing hold a solution of an active ingredient with a high water content, without impairing the properties and stability a drug or worsening the dosage characteristics or texture. The hard capsules of the present invention can be efficiently produced, in that the liquid nature of their contents enables the capsules to be filled with ease and the volume of the contents are easily adjusted.

## Claims

1. A hard capsule filled with a solution containing an active ingredient, wherein the shell of the capsule is made of a material containing a cellulose derivative, and the solution has a water content (w) of 10% < w ≤ 50%, and a water activity (a) of 0.60 ≤ a ≤ 0.90.

2. The hard capsule as described in claim 1, wherein the cellulose derivative is one or more compounds selected from the group consisting of hydroxypropyl methylcellulose, methylcellulose, hydroxypropyl cellulose, and hydroxyethyl cellulose.

3. The hard capsule as described in claim 1 or 2, wherein the solution is an aqueous extract of a medicinal herb, an animal- or plant-originating substance, or fermented matter.
